# EUROPEAN PATENT SPECIFICATION

(11) **EP 3 466 414 B1**
(45) Date of publication and mention of the grant of the patent: **07.04.2021**
(21) Application number: 16902702.6
(22) Date of filing: 26.05.2016
(51) Int. Cl.: A61K 31/122, A61P 25/24, A61K 9/00

(54) **USE OF NOOTKATONE**
VERWENDUNG VON NOOTKATON
UTILISATION DE NOOTKATONE

(43) Date of publication of application: 10.04.2019
(73) Proprietor: Institute of Tropical Bioscience and Biotechnology, Chinese Academy of Tropical Agricultural Sciences, Haikou, Hainan 571101 (CN)
(72) Inventor: DAI, Haofu, Haikou Hainan 571101 (CN); MEI, Wenli, Haikou Hainan 571101 (CN); DONG, Wenhua, Haikou Hainan 571101 (CN)
(74) Representative: Ström & Gulliksson AB
(86) International application number: PCT/CN2016/083468
(87) International publication number: WO 2017/201712

(56) References cited:
- EP-A1- 2 813 484
- CN-A- 104 162 083
- CN-A- 104 888 152
- CN-A- 104 998 205
- JP-A- 2005 330 208
- JP-A- 2007 302 572
- JP-A- 2010 280 598
- KR-U- 20110 001 860
- US-A1- 2008 085 783
- US-A1- 2011 118 359
- US-A1- 2014 271 923
- ZHENG GUANG ET AL: "Exploring the potential therapeutic mechanism of Xiao-Yao-San for major depression", 2014 IEEE INTERNATIONAL CONFERENCE ON BIOINFORMATICS AND BIOMEDICINE (BIBM), IEEE, 2 November 2014 (2014-11-02), pages 229-234, XP032721116, DOI: 10.1109/BIBM.2014.6999365 [retrieved on 2014-12-29]
- MONIKA WERNER ET AL: "Passage", PRAXIS AROMATHERAPIE: GRUNDLAGEN - STECKBRIEFE - INDIKATIONEN ; 6 TABELLEN, STUTTGART : HAUG, DE, PAGE(S) 106 - 107 , 2009, XP009517642, ISBN: 978-3-8304-7189-9 Retrieved from the Internet: URL:https://books.google.nl/books?id=aY0N3 _ApcasC&pg=PA107&lpg=PA107&dq=nootkatone+d epression&source=bl&ots=AS_jGj0jM7&sig=ACf U3U2OzcAY3pugwtNR9EA0z7xobaahHg&hl=de&sa=X &ved=2ahUKEwjR7MfptKnmAhWEiFwKHaklAtc4KBDo ATAGegQIChAB#v=onepage&q=nootkatone%20depr ession&f=false [retrieved on 2019-12-10]
- WANG, WEIXIA et al.: "Pharmacological Actions of Traditional Herbal Medicines for Improving Cognitive Dysfunction and Active Ingredients thereof", FOREIGN MEDICAL SCIENCES: TRADITIONAL CHINESE MEDICINE SECTION, vol. 27, no. 3, 31 December 2005 (2005-12-31), pages 157-161, XP009511952, ISSN: 1001-1145
- CAO, LISHA et al.: "Research Progress on Action Targets of Antidepressants", CHINA PHARMACY, vol. 25, no. 13, 31 December 2014 (2014-12-31), pages 1227-1231, XP009511937, ISSN: 1001-0408

## Description

### FIELD

The present disclosure relates to the field of pharmaceutical technology, specifically to use of nootkatone.

### BACKGROUND

Depression is a type of neurosis mainly characterized by prolonged depression, anxiety, physical discomfort, and sleep disorders. In severe cases, suicidal thoughts and behavior may occur. Studies by European and American researchers show that 15% of people in the community have depression. With the aging of China's social population and increasing social pressure, more and more studies have shown that the incidence and consultation rate of depression are on the rise.

So far, the etiology and pathogenesis of depression are still unclear, and it may be related to the decrease of the concentration of 5-HTP and norepinephrine, the neurotransmitters in the synaptic cleft of the brain. In the past decades, research on antidepressant drugs has developed rapidly, and many new types of antidepressant drugs have emerged one after another. The existing antidepressant drugs are mainly divided into three categories: monoamine oxidase inhibitors, tricyclic antidepressants and heterocyclic antidepressants. Monoamine oxidase inhibitors are the earliest antidepressants developed, which were discovered in the early 1950s. Due to the severe toxicity, they are quickly replaced by tricyclic antidepressants. Tricyclic antidepressants are currently the most widely used antidepressant drugs, including amitriptyline, doxepin, imipramine, clomipramine, and are applicable to all kinds of depression. Heterocyclic antidepressants include maprotiline, Mianserin, fluoxetine (fluorine phenoxypropanol fluoxetine, fluoxetine hydrochloride, Prozac), paroxetine, fluoochlor sertraline, fluorine aniline. Depression is a chronic disease, so taking these antidepressant drugs for a long term not only requires expensive fees, but also has strong toxic and side effects, which brings great pains to patients.

Comparing with western medicine, traditional Chinese medicine is safer. Currently, there are Chinese medicines for antidepressants such as Puyu capsule, Jieyu'anshen granules, and Jieyu pill on the market. However, they are expensive and have slow efficacies, and have not achieved satisfactory results. Therefore, there is still a need for traditional Chinese medicine preparations that are effective and inexpensive for treating depression.

Zheng Guang et al (Exploring the potential therapeutic mechanism of Xiao-Yao-San for major depression, 2014 IEEE INTERNATIONAL CONFERENCE ONBIOINFORMATICSANDBIOMEDCINE(IBM), IEEE, 2014, November 229-234) relates to the use of the Chinese medicine Xiao-Yao-San in the treatment of major depression; Werner M. (Praxis aromatherapie: grundlagen-Steckbriefe-indikationen; 6 Tabellen, Stuttgart: HAUG, DE, 2009, 106-107) discloses the use of grapefruit oil in aromatherapy for treating depression; KR2020110001860U discloses that the main extract of grapefruit seed oil is limonene and nootkatone which can promote secretion of gastric bile and is useful in lipolysis; and US2008085783A1 discloses that nootkatone is a perfume material, which gives off scents of fruits and flowers. However, none of these prior art discloses, teaches or hints that nootkatone can be used in treating or preventing depression.

### SUMMARY

In view of this, an object of the present disclosure is to provide nootkatone for use in treating and preventing depression. The nootkatone has significant curative efficacy for depression, and it is from a natural source with low price and without toxicity and side effect, which is capable to be used in preparation of related medicines.

Since it was discovered in 1960s, nootkatone has been normally used for preparing orange essence, and has been widely used in the field of tobaccos and foods. The structure formula of which is shown herein:

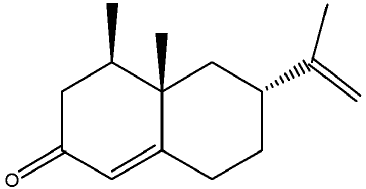

However, there is little application of nootkatone on diseases, nor reports about its use for preventing and treating depression. When screening the antidepressant drugs, the present disclosure finds that nootkatone has function of preventing and treating depression. In addition, it can be extracted from traditional Chinese herbal medicine *Alpinia oxyphylla* Miq., which is from a natural source and without toxicity and side effects. In addition, nootkatone can also be obtained by chemical synthesis, so that it has a low price and a wide range of resources.

In the pharmacodynamics test of nootkatone, the present disclosure compares nootkatone with the present positive drug Prozac in three aspects: immobility time of mouse tail suspension test, immobility time of mouse forced swimming test, and 5-HTP-induced head twitching mouse model. The results show that nootkatone has significant effects in the above three aspects and is superior to the present product Prozac.

In addition, the present disclosure also uses Hamilton Depression Scale as a criterion to verify the clinical efficacy. The results show that after 6-week of treatment, in 40 patients, 14 cases are cured, accounting for 35%; fourteen cases are significantly improved, accounting for 35%; ten cases are improved, accounting for 25%; and 2 cases have no change, accounting for 5%. No case has adverse reactions. The clinic observations show that the curative effective rate reaches 95%, showing a significant curative effect on depression and without toxicity or side effect.

On account of the pharmacodynamics and clinical effect, the present disclosure provides use of nootkatone in preparing medicines for preventing and/or treating depression. The dosage form of the medicine can be any type of oral preparations, injection preparations or external preparations in medicine field. Therein, the oral preparation can be tablets, capsules, pills, granules, decoctions, gels, ointments, distillate formulas, oral liquids, dripping pills, syrups.; the injection preparation can be injection liquids, freeze-dried powders; and the external preparation can be spray, nasal drops. The above preparations can be produced from routine pharmaceutic adjuvants in the art and reference can be made to *The Extra Pharmaceutical Necessities* (edited by Luo, Mingsheng).

Based on the above technical solutions, the present disclosure provides use of nootkatone in the manufacture of a medicament for preventing and/or treating depression, which has a remarkable curative effect on the treatment of depression and is superior to the present antidepressant drug Prozac. In addition, since it is a natural ingredient form a traditional Chinese herbal medicine and can be obtained either through extraction or through chemical synthesis, it has a wide range of sources, and it is natural, low in price, without toxicity and side effects, providing more options for patients to prevent and treat depression.

### DETAILED DESCRIPTION

The present disclosure discloses nootkatone for use in the preventing and/or treating depression.

The present disclosure will be further illustrated in conjunction with the following examples.

### Example 1: Pharmacodynamics experiment of nootkatone

One week before the experiment, normal ICR mice were housed in different cages, with Light-Dark cycle of 12h/12h, room temperature between 20 and 22°C, and free access to foods and drinks. Before the experiment, mice were subjected to qualification test by being put in a water-containing cylindrical glass container with a height of 20cm and a diameter of 12cm. The water has a depth of 12cm and temperature of 25°C. The mice were observed for 6min, and the accumulative immobility time of the mice in the last 4 min was recorded. The mice having an accumulative immobility time from 100 to 190s were the qualified mice. The so-called immobility meant that the mice stopped struggling in the water, or in state of floating and only the limbs moved slightly to keep their heads above the water. 100 qualified normal ICR mice were randomly divided into 5 groups, i.e., a blank control group (distilled water), a positive drug control group (Prozac, 20mg/Kg), a large-dose group of nootkatone (2mg/Kg), a medium-dose group of nootkatone (1.5mg/Kg) and a small-dose group of nootkatone (1mg/Kg). In all the groups, both the male and the female accounted for half. All the experiments were carried out between 1:00 pm and 5:00 pm, and the mice in each group were respectively administered with distilled water, Prozac and nootkatone by intragastric gavage according to the dosages. The administration was performed once a day and lasted for 14 days. The body weights of the mice before the administration, one week after the administration and 2 weeks after the administration were recorded, and the fur appearance of the mice were observed. After the last administration, behavior tests were performed on the ICR mice, including a tail suspension test of the mice, a forced swimming test of the mice and a 5-HTP induced head-twitching mice model.

### 1. Tail suspension test

One and half an hour after the last administration, the mice were subjected to tail suspension test. The tail of a mouse was fastened at 2cm position with wooden clips, and the mouse was hung upside down in a 12×27×27cm box, with the head approximately 5cm from the bottom. The mouse was hung for 6min, and the accumulative immobility time in the last 4min was recorded. The results of the data analysis were shown in Table 1.

**Table 1 Effect of nootkatone on immobility time of mice in tail suspension test**

| Group | Dosage (Mg/Kg) | Immobility Time (S) |
|---|---|---|
| Blank Control Group (Distilled Water) | 0 | 151.1±20.1 |
| Positive Control Group (Prozac) | 20 | 87.9±22.0** |
| Large-dose Group of Nootkatone | 2 | 75.1±19.6** |
| Medium-dose Group of Nootkatone | 1.5 | 80.2±21.6** |
| Small-dose Group of Nootkatone | 1 | 84.0±17.6** |

| | | |
|---|---|---|
| **P<0.01 | | |

### 2. Forced swimming test

One and half an hour after the last administration, the mice were subjected to forced swimming test. The mice were placed in a glass container and observed for 6min, and the accumulative immobility time in the last 4min was recorded. The results of the data analysis were shown in Table 2.

**Table 2 Effects of nootkatone on immobility time of mice in forced swimming test**

| Group | Dosage (mg/Kg) | Immobility Time (s) |
|---|---|---|
| Blank Control Group (Distilled Water) | 0 | 148.1±18.9 |
| Positive Control Group (Prozac) | 20 | 80.9±20.4** |
| Large-dose Group of Nootkatone | 2 | 66.1±21.6** |
| Medium-dose Group of Nootkatone | 1.5 | 70.2±22.3** |
| Small-dose Group of Nootkatone | 1 | 74.0±26.9** |

| | | |
|---|---|---|
| **P<0.01 | | |

### 3. 5-HTP induced head twitching mouse model

Half an hour after the last administration, 150mg/Kg of 5-HTP was intraperitoneal injected and mice were placed in cage respectively. The head-twitching numbers of the mice in 20min were recorded and the results of data analysis were shown in Table 3.

**Table 3 Effects of nootkatone on 5-HTP induced head twitching mice model**

| Group | Dosage (mg/Kg) | Head-Twitching Number (Time) |
|---|---|---|
| Blank Control Group (Distilled Water) | 0 | 34.1±18.1 |
| Positive Control Group (Prozac) | 20 | 57.9±10.4** |
| Large-dose Group of Nootkatone | 2 | 63.1±22.5** |
| Medium-dose Group of Nootkatone | 1.5 | 60.2±25.1** |
| Small-dose Group of Nootkatone | 1 | 58.0±26.2** |

| | | |
|---|---|---|
| **P<0.01 | | |

### 4. Conclusions

As shown in Table 1, Table 2 and Table 3, when comparing with the blank control group, both nootkatone and positive drug Prozac obviously decreased the immobility times in tail suspension test and forced swimming test of mice in experimental group, and obviously increased the head twitching number of the 5-HTP induced head twitching mouse model. The efficacy of nootkatone was better than that of Prozac. The results demonstrated that nootkatone has a function of decreasing despair behaviors of mice.

By observing the whole appearance of mice, there was no significant difference between the weight of mice before administration, one week after administration and 2 weeks after administration, and the fur was lustrous and there were no significant differences, indicating that neither nootkatone nor the positive drug Prozac has toxicity.

### Example 2: clinical efficacy observation

Forty patients were selected, wherein 20 were male and 20 were female, and their disease durations were from 1 month to 2 years. Diagnosis criteria of depression that conformed to *Chinese Classification and Diagnostic Criteria of Mental Disorders* scored 8 according to the first 17 items in the Hamilton Depression Rating Scale (HAM-D) shown in Table 4. First-onset patients, or patients that had not taken antidepressants and antipsychotics and had not been subjected to electric shock therapy over the past week, and patients with depressive episodes of unipolar affective disorder, aged 20-50 years. Patients having depressive disorder caused by bipolar affective disorder and other causes, severe suicidal tendency, and severe abnormalities in laboratory tests (pregnant or lactating women) were eliminated.

**Table 4 Hamilton Depression Rating Scale (HAM-D)**

| | Item | Standard for Evaluation | Score | | | | |
|---|---|---|---|---|---|---|---|
| | | | Absent | Mild | Moderate | Severe | Very Severe |
| 1 | Depressed Mood | 0 = Absent | 0 | 1 | 2 | 3 | 4 |
| | | 1 = These feeling states indicated only on questioning. | | | | | |
| | | 2 = These feeling states spontaneously reported verbally. | | | | | |
| | | 3 = Communicates feeling states non-verbally, i.e. through facial expression, posture, voice and tendency to weep | | | | | |
| | | 4 = Patient reports virtually only these feeling states in his/her spontaneous verbal and non-verbal communication. | | | | | |
| 2 | Feelings of Guilt | 0 = Absent. | 0 | 1 | 2 | 3 | 4 |
| | | 1 = Self reproach, feels he/she has let people down. | | | | | |
| | | 2 = Ideas of guilt or rumination over past errors or sinful deeds. | | | | | |
| | | 3 = Present illness is a punishment. Delusions of guilt. | | | | | |
| | | 4 = Hears accusatory or denunciatory voices and/or experiences threatening visual hallucinations | | | | | |
| 3 | Suicide | 0 = Absent | 0 | 1 | 2 | 3 | 4 |
| | | 1 = Feels life is not worth living | | | | | |
| | | 2 = Wishes he/she were dead, or any thoughts of possible death to self | | | | | |
| | | 3 = Suicide, ideas or half-hearted attempts | | | | | |
| | | 4 = Attempts at suicide | | | | | |
| 4 | Insomnia (Early) | 0 = No difficulty falling asleep | 0 | 1 | 2 | - | - |
| | | 1 = Complains of occasional difficulty falling asleep; i.e. more than half an hour (pay attention to the usual time for patients to fall asleep) | | | | | |
| | | 2 = Complains of nightly difficulty falling asleep | | | | | |
| 5 | Insomnia (Middle) | 0 = No difficulty | 0 | 1 | 2 | - | - |
| | | 1 = Patient complains of being restless and disturbed during the night | | | | | |
| | | 2 = Waking during the night (before 12:0 0 p.m., excepting for voiding) | | | | | |
| 6 | Insomnia (Late) | 0 = Absent | 0 | 1 | 2 | - | - |
| | | 1 = Waking in early hours of the morning (1 hour earlier than usual) but goes back to sleep | | | | | |
| | | 2 = Unable to fall asleep gain if he/she wakes in early hours | | | | | |
| 7 | Work and Activities | 0 = No difficulty. | 0 | 1 | 2 | 3 | 4 |
| | | 1 = These feeling states indicated only on questioning. | | | | | |
| | | 2 = Loss of interest in activity, hobbies or work - either directly reported by the patient or indirect in listlessness, indecision and vacillation (feels he/she has to push self to work or activities). | | | | | |
| | | 3 = The patient spend less than three hours a day on labor or entertainment. | | | | | |
| | | 4 = Stopped working because of present illness. The patient engages in no activities, or the patient fails to perform routine chores unassisted without help of other people. | | | | | |
| 8 | Retardation | 0 = Normal speech and thought. | 0 | 1 | 2 | 3 | 4 |
| | | 1 = Slight retardation during the interview. | | | | | |
| | | 2 = Obvious retardation during the interview. | | | | | |
| | | 3 = Interview difficult. | | | | | |
| | | 4 = Complete stupor. | | | | | |
| 9 | Agitation | 0 = None. | 0 | 1 | 2 | 3 | 4 |
| | | 1 = Fidgetiness during the interview. | | | | | |
| | | 2 = Obviously unset, or performs numerous petty actions. | | | | | |
| | | 3 = Cannot sit quietly, or stand up during the interview. | | | | | |
| | | 4 = Hand wringing, nail biting, hair-pulling, biting of lips. | | | | | |
| 10 | Anxiety (Psychic) | 0 = No difficulty. | 0 | 1 | 2 | 3 | 4 |
| | | 1 = These feeling states indicated only on questioning. | | | | | |
| | | 2 = Spontaneously express. | | | | | |
| | | 3 = Apprehensive attitude apparent in face or speech. | | | | | |
| | | 4 = Obvious fears. | | | | | |
| 11 | Anxiety (Somatic) | Physiological concomitants of anxiety, i ncluding dry mouth, abdomina 1 distension, diarrhea, hiccup, abdominal angina, palpitations, headaches, Hyperventilation and sighing, urinary frequency,. | 0 | 1 | 2 | 3 | 4 |
| | | 0 = Absent. | | | | | |
| | | 1 = Mild. | | | | | |
| | | 2 = Moderate, there are certainly the above symptoms. | | | | | |
| | | 3 = Severe, the above symptoms are serious, and affect life or need to be dealt with. | | | | | |
| | | 4 = Seriously affect life and activities. | | | | | |
| 12 | Somatic Symptoms (Gastrointe stinal) | 0 = None. | 0 | 1 | 2 | - | - |
| | | 1 = Loss of appetite but eating without staff encouragement. | | | | | |
| | | 2 = Difficulty eating without staff urging. Requests or requires laxatives or medication for bowels or medication for gastro-intestinal symptoms. | | | | | |
| 13 | Somatic Symptoms (General) | 0 = None. | 0 | 1 | 2 | - | - |
| | | 1 = Heaviness in limbs, back or head. Backaches, headaches, muscle aches. Loss of energy and fatigability. | | | | | |
| | | 2 = Clear-cut symptoms. | | | | | |
| 14 | Genital Symptoms | (Symptoms such as loss of libido, menstrual disturbances) | 0 | 1 | 2 | - | - |
| | | 0 = Absent. | | | | | |
| | | 1 = Mild. | | | | | |
| | | 2 = Severe. | | | | | |
| | | Not sure, or that the item is not suitable for the person to be rated (not included in the total score). | | | | | |
| 15 | Hypochondriasis | 0 = Not present. | 0 | 1 | 2 | 3 | 4 |
| | | 1 = Pay excessively attention to oneself (bodily) . | | | | | |
| | | 2 = Preoccupation with health. | | | | | |
| | | 3 = Hypochondriacal delusions, and frequent complaints, requests for help. | | | | | |
| | | 4 = Hypochondriacal delusions with hallucination. | | | | | |
| 16 | Loss of Weight | Rate either A) or B): | 0 | 1 | 2 | - | - |
| | | A) According to the patient: | | | | | |
| | | 0 = No weight loss. | | | | | |
| | | 1 = Probable weight loss associated with present illness. | | | | | |
| | | 2 = Definite (according to patient) weight loss. | | | | | |
| | | B) According to weekly measurements: | | | | | |
| | | 0 = Less than 0.5kg weight loss in week. | | | | | |
| | | 1 = Greater than 0.5kg weight loss in week. | | | | | |
| | | 2 = Greater than 1kg weight loss in week. | | | | | |
| 17 | Insight | 0 = Acknowledges being depressed and i11. | 0 | 1 | 2 | 3 | 4 |
| | | 1 = Acknowledges illness but attributes cause to bad food, climate, overwork, virus, need for rest. | | | | | |
| | | 2 = Denies being ill at all. | | | | | |

Comment: 0 to 4 in the items of Standard for Evaluation and Point referred to the scores, and the standard for evaluation is: the total score <7 means normality; the total score between 7 and 17 means possible depression; the total score between 17 and 24 means definite depression; and the total score >24 means severe depression.

Nootkatone preparation was used to treat the 40 patients, and the administration manner was: orally taking, 1.5mg nootkatone monomer/Kg/ time, twice a day, and 6 weeks were one course of treatment. The patients were observed in the course of treatment for any adverse reaction. After 1 course of treatment, reduction rate of HAM-D was used to measure the curative efficacy of the traditional Chinese medicine on patients, reduction rate of HAM-D = (total scores before the treatment - total scores after the treatment)/total scores before the treatment × 100%.

The standard of curative efficacy evaluation is hereinafter:
cure: reduction rate of HAMD is >75%;
significantly improved: the reduction rate of HAMD is from 51% to 75%;
improved: reduction rate of HAMD is from 25% to 50%;
invalid: reduction rate of HAMD is <25%.

After 6-week treatment, in the 40 patients, 14 cases were cured, accounting for 35%; 14 cases were significantly improved, accounting for 35%; 10 cases were improved, accounting for 25%; and 2 cases were invalid, accounting for 5%, and no case has adverse reactions. The clinical efficacy observation showed that the curative effective rate of nootkatone reached 95% in the patients, indicating a significant curative effect on depression and without toxicity and side effect.

## Claims

1. Nootkatone for use as a medicament in the prevention or treatment of depression.

2. Nootkatone for use according to claim 1, wherein the medicament is an oral preparation, an injection preparation or an external preparation.

3. Nootkatone for use according to claim 2, wherein the oral preparation is a tablet, a capsule, a pill, granules, a decoction, a gel, an ointment, a distillate formula, an oral liquid, a dripping pill or a syrup.

4. Nootkatone for use according to claim 2, wherein the injection preparation is an injection liquid or a freeze-dried powder.

5. Nootkatone for use according to claim 2, wherein the external preparation is a spray or nasal drops.

## Patentansprüche

1. Nootkaton zur Verwendung als Medikament bei der Prävention oder Behandlung von Depressionen.

2. Nootkaton zur Verwendung nach Anspruch 1, wobei das Medikament ein orales Präparat, ein Injektionspräparat oder ein externes Präparat ist.

3. Nootkaton zur Verwendung nach Anspruch 2, wobei das orale Präparat eine Tablette, eine Kapsel, eine Pille, Granulat, ein Dekokt, ein Gel, eine Salbe, eine Destillatformel, eine orale Flüssigkeit, ein Tropfmedikament oder ein Sirup ist.

4. Nootkaton zur Verwendung nach Anspruch 2, wobei das Injektionspräparat eine Injektionsflüssigkeit oder ein gefriergetrocknetes Pulver ist.

5. Nootkaton zur Verwendung nach Anspruch 2, wobei das externe Präparat ein Spray oder Nasentropfen ist/sind.

## Revendications

1. Nootkatone pour une utilisation comme médicament dans la prévention ou le traitement d'une dépression.

2. Nootkatone pour une utilisation selon la revendication 1, dans laquelle le médicament est une préparation orale, une préparation pour injection ou une préparation externe.

3. Nootkatone pour une utilisation selon la revendication 2, dans laquelle la préparation orale est un comprimé, une capsule, une pilule, des granulés, une décoction, un gel, une pommade, une formule de distillat, un liquide oral, une pilule goutte-à-goutte ou un sirop.

4. Nootkatone pour une utilisation selon la revendication 2, dans laquelle la préparation pour injection est un liquide pour injection ou une poudre lyophilisée.

5. Nootkatone pour une utilisation selon la revendication 2, dans laquelle la préparation externe est une pulvérisation ou des gouttes nasales.
